# DEMANDE DE BREVET EUROPEEN

(11) **EP 1 967 150 A1**
(43) Date de publication de la demande: **10.09.2008**
(21) Numéro de dépôt: 08152498.5
(22) Date de dépôt: 07.03.2008
(51) Int. Cl.: A61B 17/70

(54) **Dispositif d'ancrage vertébral par clou intrapédiculaire**

(30) Priorité: 09.03.2007 FR 0753751
(71) Demandeur: Roussouly, Pierre, 69450 Saint Cyr au Mont d'Or (FR)
(72) Inventeur: Roussouly, Pierre, 69450 Saint Cyr au Mont d'Or (FR)
(74) Mandataire: Poncet, Jean-François

(57) **Abrégé**

Un ancrage vertébral selon l'invention est réalisé par l'assemblage de deux pièces d'ancrage (1, 1a), comportant chacune un clou intrapédiculaire (5, 5a) engagé dans l'un des pédicules (3d, 3e), et reliées l'une à l'autre par une liaison rigide (4) constituée par des branches transversales de liaison (6, 6a) solidarisées l'une à l'autre. L'ensemble est rigide et réalise un arc-boutement des deux clous intrapédiculaires (5, 5a), assurant une grande stabilité sans dégrader l'os.

## Description

La présente invention concerne les dispositifs d'ancrage vertébral permettant l'ostéosynthèse du rachis.

L'ostéosynthèse du rachis est réalisée généralement par des systèmes permettant de relier rigidement l'une à l'autre au moins deux vertèbres du rachis. Les systèmes comportent des dispositifs d'ancrage vertébral se fixant chacun de manière rigide à une vertèbre, et des plaques ou des tiges de réduction reliant l'un à l'autre plusieurs dispositifs d'ancrage vertébral fixés à des vertèbres différentes.

Les dispositifs d'ancrage vertébral actuellement les plus utilisés sont essentiellement de deux types : les crochets et les vis intrapédiculaires.

Dans la plupart des cas, ces ancrages connus donnent satisfaction, lorsque l'os de la vertèbre est de bonne qualité. En effet, un crochet peut venir s'engager derrière une portion résistante de l'os, tandis qu'une vis intrapédiculaire peut venir s'engager dans le canal intérieur d'un pédicule et est alors entourée d'os cortical résistant.

Mais dans le cas d'une vertèbre affaiblie, par exemple par ostéoporose, notamment lorsque les forces de réduction sont importantes, appliquant d'une vertèbre à l'autre une contrainte mécanique importante, la stabilité de l'implant est menacée, avec un risque d'arrachement. Dans les techniques connues, on a alors besoin de multiplier les prises osseuses pour chaque ancrage, mais cela complique la pose, augmente le matériel qu'il faut implanter, et agresse encore plus l'os déjà affaibli.

Le document US 5,306,275 A décrit un ancrage par vis pédiculaire (figure 13) stabilisée par une coiffe d'ancrage dont plusieurs dents pénètrent dans le corps vertébral. La vis constitue l'ancrage principal et comporte un trou transversal de passage d'une tige de réduction.

Le document US 2005/0216004 A1 décrit, dans un mode de réalisation, un système d'ancrage conformé pour s'ancrer par voie antérieure à un corps vertébral. Ce système d'ancrage comprend une plaque-agrafe cintrée pour suivre la surface du corps vertébral, avec au moins une pointe d'agrafe courte et deux trous taraudés pour recevoir deux moyens d'ancrage. Le moyen d'ancrage principal est une vis à os engagée dans un premier trou taraudé et dont la tête a un trou transversal de passage d'une tige flexible de réduction ; un moyen d'ancrage complémentaire peut être un clou à tête filetée vissée dans l'autre des trous taraudés. Le clou d'ancrage complémentaire et la vis d'ancrage principal sont conformés pour être implantés dans le corps vertébral. Ce mode de réalisation n'est pas prévu ni adapté pour une implantation par voie postérieure avec des ancrages pédiculaires. Il nécessite en outre l'assemblage d'au moins trois pièces.

On connaît également des systèmes comportant une plaque interpédiculaire que l'on fixe à une vertèbre par deux vis pédiculaires engagées respectivement dans l'un et l'autre des pédicules d'une même vertèbre. Ces systèmes ne comportent pas de liaison forte entre les vis et la plaque pédiculaire, et ne procurent pas un véritable couplage des vis, qui peuvent osciller par rapport à la plaque.

Les vis pédiculaires constituent des éléments relativement agressifs qui, par leur filet, réalisent des amorces de rupture dans l'os dans lequel elles sont engagées. On constate encore une stabilité insuffisante et un risque d'arrachement dans le cas d'os affaibli par ostéoporose.

Le problème proposé par la présente invention est de concevoir un nouveau dispositif d'ancrage vertébral permettant d'assurer un ancrage efficace et présentant une plus faible agressivité vis-à-vis de l'os, évitant pour cela l'utilisation de vis.

L'idée qui est à la base de l'invention est d'utiliser un ancrage principal par un clou pédiculaire.

L'homme du métier est a priori dissuadé d'envisager un ancrage principal par clou pédiculaire, considérant qu'un clou peut être arraché et réalise habituellement un ancrage moins résistant qu'une vis, conduisant donc à un manque de stabilité.

Par contre, l'invention permet d'obtenir une stabilité satisfaisante en réalisant un accouplement particulier du clou pédiculaire à des moyens d'ancrage complémentaires, assurant alors une bonne résistance à l'arrachement.

Ainsi, pour résoudre ce problème, l'invention propose un dispositif d'ancrage vertébral, comprenant un moyen d'ancrage principal et un moyen d'ancrage complémentaire conformés pour s'ancrer chacun par voie postérieure respectivement à une vertèbre et pour être connectés mécaniquement l'un à l'autre, dans lequel :
- le moyen d'ancrage principal est une pièce rigide, comportant un clou pédiculaire à tête proximale et à tige axiale conformée pour être engagée en force dans un canal intérieur d'un pédicule de vertèbre, comportant une branche transversale proximale de liaison, et comportant des moyens de connexion pour sa connexion à une tige de réduction apte à relier plusieurs dispositifs d'ancrage vertébral,
- la branche transversale de liaison du moyen d'ancrage principal est munie de moyens de solidarisation pour sa solidarisation au moyen d'ancrage complémentaire,
- le moyen d'ancrage complémentaire est conformé pour s'opposer à l'arrachement axial de la tige de clou pédiculaire du moyen d'ancrage principal hors du pédicule de vertèbre.

Le clou pédiculaire à branche transversale de liaison est une structure rigide qui assure une liaison directe, rigide et mécaniquement résistante entre la vertèbre et une tige de réduction. II forme ainsi le véritable moyen d'ancrage de la tige de réduction sur la vertèbre. C'est pourquoi l'invention le désigne par l'expression "moyen d'ancrage principal".

Pour assurer un ancrage mécaniquement résistant dans la vertèbre, la tige axiale du clou pédiculaire est conformée pour être engagée en force dans le canal intérieur d'un pédicule de la vertèbre concernée, et pour se déployer jusque dans l'os du corps vertébral, tout en faisant en sorte que son extrémité reste à l'intérieur du corps vertébral. Or les dimensions en diamètre des canaux intérieurs de pédicule, et la taille des corps vertébraux, varient en fonction des vertèbres considérées et des individus. Il n'est ainsi pas possible de déterminer une forme unique de clou pédiculaire pouvant convenir pour tous les ancrages. II faut choisir un clou pédiculaire dont la tige est conformée de façon appropriée pour un engagement efficace. En pratique, on prévoit une gamme de clous pédiculaires comportant diverses longueurs et divers diamètres de tige. Les longueurs sont comprises entre 45 mm environ et 50 mm environ. Les diamètres sont compris entre 5,5 mm environ et 7,5 mm environ. Pour une implantation, on choisit, parmi la gamme, le clou pédiculaire dont la forme de tige permet sa pénétration complète avec un engagement correct en force, sans excès d'effort, dans le canal intérieur du pédicule considéré et dans le corps vertébral. Ainsi, la forme de la tige du clou pédiculaire, notamment sa longueur et son diamètre, est définie par référence à la forme du canal intérieur du pédicule considéré, notamment son diamètre, et par référence à la taille du corps vertébral.

La combinaison de deux moyens d'ancrage complémentaires assure une bonne résistance à l'arrachement du clou pédiculaire engagé dans le canal intérieur d'un pédicule de vertèbre.

Simultanément, le clou constitue, dans le pédicule, une structure lisse et peu agressive, qui évite les risques de détérioration d'un os déjà affaibli par l'ostéoporose, et qui améliore la tenue aux efforts transversaux de cisaillement.

En pratique, la tige du clou pédiculaire peut avoir un corps tronconique dont le diamètre va en se réduisant progressivement à partir de la tête à laquelle se relie la branche transversale de liaison. La forme tronconique est adaptée à la pénétration en force dans le canal correspondant du pédicule, et optimise la surface d'appui du clou sur l'os cortical.

La liaison des vertèbres par une tige de réduction nécessite de prévoir des moyens de connexion pour connecter la tige de réduction et le dispositif d'ancrage vertébral. Pour cela, on peut avantageusement prévoir des moyens de connexion sur chaque moyen d'ancrage principal, et par exemple prévoir que la tête proximale comporte un ou plusieurs alésages taraudés pour l'insertion d'une tige filetée de connexion.

En pratique, la tête proximale peut comporter un alésage taraudé axial, débouchant à l'opposé de la tige du clou pédiculaire, pour l'insertion d'une tige filetée de connexion. De préférence, la tête proximale comporte en outre un alésage taraudé latéral, débouchant à l'opposé de la branche transversale de liaison, avantageusement selon une orientation de 90° ou 120° par rapport à l'axe de la tige du clou pédiculaire, pour l'insertion d'une tige filetée de connexion qui est alors orientée latéralement. On peut ainsi, lors de l'implantation, choisir la meilleure orientation de la tige filetée de connexion pour placer au mieux la tige de réduction par rapport au dispositif d'ancrage, soit en position postérieure, soit en position latérale.

Selon un mode de réalisation avantageux, la branche transversale de liaison est galbée, de façon à épouser le profil de l'arc postérieur de la vertèbre sur laquelle elle doit être implantée.

Dans ce cas, les moyens de solidarisation peuvent comprendre un trou dont l'axe est généralement parallèle à l'axe de la tige du clou pédiculaire. On peut ainsi solidariser aisément deux branches transversales de liaison en engageant un ensemble vis-écrou dans les trous des deux branches transversales de liaison mis en correspondance. L'ensemble vis-écrou est aisément adaptable après la pose des clous pédiculaires sur le patient.

Un dispositif d'ancrage vertébral selon l'invention peut alors avantageusement comporter deux moyens d'ancrage principaux, éventuellement identiques, solidarisés l'un à l'autre de façon rigide par un ensemble vis-écrou engagé dans des trous correspondants des branches transversales de liaison respectives orientées l'une vers l'autre et venant à recouvrement partiel l'une de l'autre, les tiges des clous pédiculaires étant orientées selon deux orientations convergentes se rapprochant l'une de l'autre vers leurs extrémités libres en suivant l'orientation naturelle des pédicules.

L'invention profite ainsi de l'orientation naturelle divergente des pédicules d'une même vertèbre, en orientant les clous dans des directions non parallèles qui assurent, du fait de la solidarisation par les branches transversales de liaison, une liaison mécanique rigide et un arc-boutement qui s'oppose à toutes possibilités d'arrachement.

Selon un autre mode de réalisation avantageux, les moyens de solidarisation du moyen d'ancrage principal peuvent comprendre une rainure transversale externe.

Dans ce cas, le moyen d'ancrage complémentaire peut être une bride apte à entourer à la fois un relief osseux de la vertèbre et la branche transversale de liaison du moyen d'ancrage principal en s'engageant dans la rainure transversale externe.

Selon un autre mode de réalisation, la branche transversale de liaison du moyen d'ancrage principal est une tige précintrée monobloc ou rapportée, apte à être connectée à une vis pédiculaire pour assurer une ostéosynthèse intervertébrale courte.

D'autres objets, caractéristiques et avantages de la présente invention ressortiront de la description suivante de modes de réalisation particuliers, faite en relation avec les figures jointes, parmi lesquelles :
- la figure 1 est une vue de dessus d'un moyen d'ancrage principal selon un mode de réalisation de la présente invention ;
- la figure 2 est une vue de face postérieure du moyen d'ancrage principal de la figure 1 ;
- la figure 3 est une vue de dessus en coupe montrant l'implantation d'un dispositif d'ancrage vertébral selon l'invention, sur une vertèbre ;
- la figure 4 est une vue de côté d'un moyen d'ancrage principal selon un second mode de réalisation de l'invention ;
- la figure 5 illustre la stabilisation du moyen d'ancrage principal de la figure 4 sur une vertèbre à l'aide d'un moyen d'ancrage complémentaire de type bride ;
- la figure 6 illustre une variante de l'implantation du moyen d'ancrage principal de la figure 4 ; et
- les figures 7 et 8 illustrent un dispositif d'ancrage selon un autre mode de réalisation de l'invention.

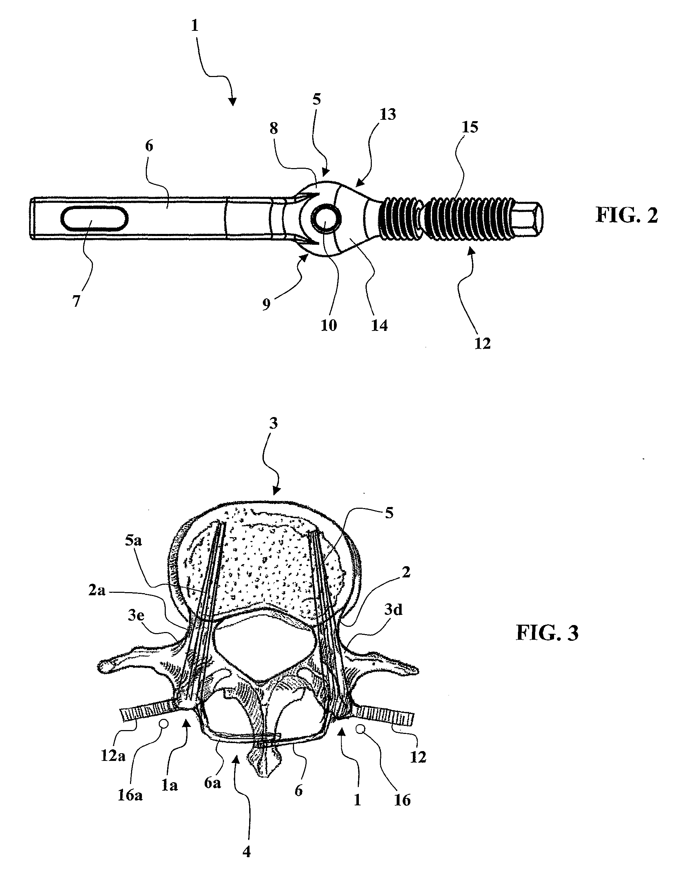

Dans le mode de réalisation illustré sur la figure 3, un dispositif d'ancrage vertébral selon l'invention comprend deux moyens d'ancrage principaux 1 et 1 a conformés pour s'ancrer chacun, par voie postérieure, respectivement sur un premier pédicule 2 et sur un second pédicule 2a d'une vertèbre 3, et pour être connectés mécaniquement l'un à l'autre par une liaison mécanique rigide 4.

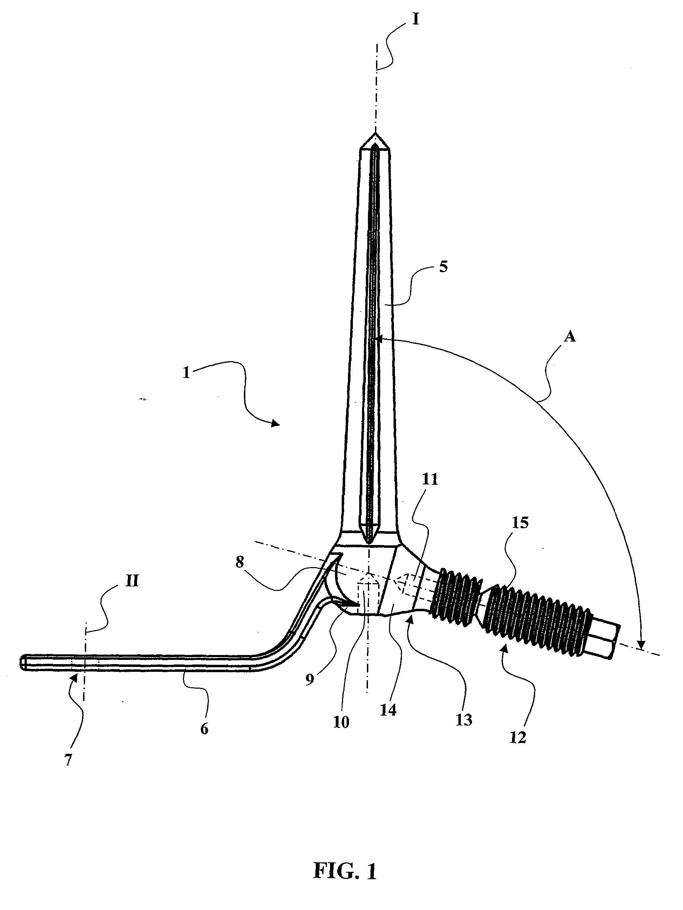

On considère maintenant les figures 1 et 2 qui illustrent de façon plus détaillée la structure des moyens d'ancrage principaux 1 ou 1a de la figure 3.

Le moyen d'ancrage principal 1 est une pièce mécaniquement rigide en forme générale de L, avec une première partie axiale constituant un clou pédiculaire à tête 8 et à tige axiale 5 conformée pour être engagée en force dans un pédicule respectif (2 ou 2a sur la figure 3), et comportant une branche transversale de liaison 6 en forme générale de bande.

La tige 5 du clou pédiculaire a une longueur de 45 mm environ à 50 mm environ, choisie pour être compatible avec la longueur d'un canal intérieur de pédicule de la vertèbre 3 considérée. La tige 5 du clou pédiculaire a une section transversale dont le diamètre est de 5, 5 mm environ à 7,5 mm environ, choisi pour être compatible avec la section transversale dudit canal intérieur de pédicule. La section transversale de la tige 5 peut être circulaire. En alternative, pour assurer en outre un effet anti-rotation, la section transversale de la tige 5 peut être non circulaire, par exemple quadrangulaire.

La branche transversale de liaison 6 comporte des moyens de solidarisation pour sa solidarisation à la branche transversale d'un autre moyen d'ancrage principal. Dans la réalisation illustrée sur les figures, les moyens de solidarisation comprennent un trou 7 dont l'axe II est généralement parallèle à l'axe I de la tige 5 du clou pédiculaire. Le trou 7 peut être oblong, allongé dans le sens de la longueur de la branche transversale de liaison 6, pour permettre un réglage de l'entre-axe entre les tiges 5 et 5a des clous pédiculaires des deux moyens d'ancrage principaux 1 et 1 a.

La tige 5 du clou pédiculaire a un corps tronconique dont le diamètre va en se réduisant progressivement à partir de la tête 8 à laquelle se relie la branche transversale de liaison 6.

La tête 8 comporte une surface externe 9 sphérique, un alésage taraudé axial 10 débouchant à l'opposé de la tige 5 du clou pédiculaire, et un alésage taraudé latéral 11 débouchant à l'opposé de la branche transversale de liaison 6, avantageusement selon une orientation A de 90° ou 120° par rapport à l'axe I de la tige 5 du clou pédiculaire.

Les alésages 10 et 11 font partie des moyens de connexion, permettant l'insertion d'une tige filetée de connexion 12.

La tige filetée de connexion 12 comporte un premier tronçon fileté apte à se visser dans l'un des alésages taraudés 10 ou 11 de la tête 8, une portion centrale 13 à portée extérieure conique 14 pour la stabilisation d'une tige de réduction, et un second tronçon fileté 15 pour la fixation de la tige de réduction 16 ou 16a (figure 3) par un adaptateur non représenté.

La portion centrale 13 de tige filetée de connexion 12 comporte une portée intérieure sphérique s'adaptant sur la surface externe 9 sphérique de la tête 8, pour sa stabilisation.

La branche transversale de liaison 6 est galbée, comme illustré sur la figure 1, de façon à épouser le profil de l'arc postérieur d'une vertèbre sur laquelle elle doit être implantée.

Dans la réalisation illustrée sur la figure 3, les moyens d'ancrage principaux 1 et 1a sont identiques. Ils sont solidarisés l'un à l'autre de façon rigide par un ensemble vis-écrou engagé dans les trous correspondants (le trou 7 de la figure 2) des branches transversales de liaison respectives 6 et 6a orientées l'une vers l'autre et venant à recouvrement partiel l'une de l'autre. Les tiges 5 et 5a des clous pédiculaires sont orientées selon deux orientations convergentes se rapprochant l'une de l'autre vers leurs extrémités libres, comme on le voit sur la figure 3, suivant en cela l'orientation naturelle des deux pédicules 3d et 3e de la vertèbre 3. Les tiges filetées de connexion 12 et 12a sont adaptées chacune pour recevoir des adaptateurs pour la solidarisation d'une tige de réduction respective telle que les tiges 16 et 16a se développant dans le plan perpendiculaire au plan de la figure 3, pour relier au moins deux vertèbres l'une à l'autre. Les adaptateurs ne sont pas représentés, mais peuvent être de tous types habituellement utilisés pour relier un dispositif d'ancrage à une tige de réduction.

Les tiges 5 et 5a des clous pédiculaires constituent des éléments d'ancrage relativement peu agressifs pour l'os des pédicules 3d et 3e, et leur arc-boutement grâce à la liaison mécanique rigide 4 permet d'assurer un ancrage efficace s'opposant aux risques d'arrachement, de sorte que la stabilité du dispositif d'ancrage est satisfaisante dans la plupart des cas.

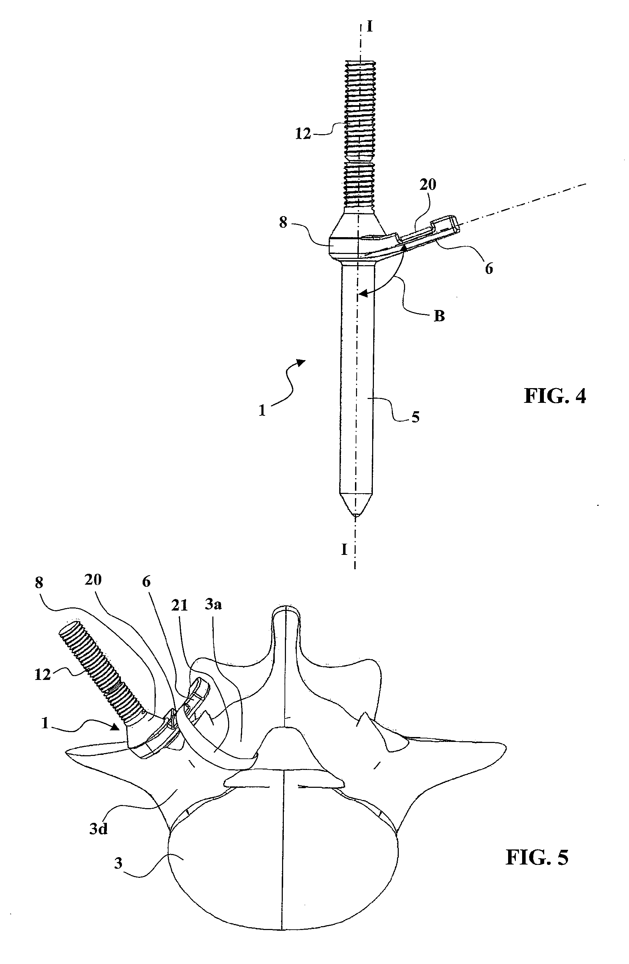

On considère maintenant les figures 4 à 6, qui illustrent un second mode de réalisation du dispositif d'ancrage selon l'invention.

Dans ce second mode de réalisation, le moyen d'ancrage principal 1, de type clou pédiculaire, est stabilisé par un moyen d'ancrage complémentaire 21 de type bride.

La structure du moyen d'ancrage principal 1 est bien visible sur la figure 4, où l'on retrouve une structure constituée d'une pièce mécaniquement rigide avec une première partie axiale constituant une tige axiale 5 de clou pédiculaire conformée pour être engagée en force dans un pédicule de vertèbre, avec une branche transversale de liaison 6 en forme générale de bande, et avec une tige filetée de connexion 12, solidaire de la tête 8, et orientée à l'opposé de la tige 5 de clou pédiculaire.

La branche transversale de liaison 6 est avantageusement orientée selon une orientation B d'environ 110° par rapport à l'axe I-I de la tige 5 du clou pédiculaire.

D'autre part, la branche transversale de liaison 6 comprend, comme moyen de solidarisation du moyen d'ancrage principal 1, une rainure transversale externe 20, ménagée sur la face de branche transversale de liaison 6 qui est orientée en direction de la tige filetée de connexion 12.

On considère maintenant la figure 5, qui illustre une première possibilité de stabilisation du moyen d'ancrage principal 1 de la figure 4, sur une vertèbre 3. Cette première possibilité de stabilisation peut être utilisée dans les cas où l'os est de mauvaise qualité, en particulier dans le cas de l'ostéoporose du sujet âgé. On renforce alors la stabilité du clou pédiculaire du moyen d'ancrage principal 1 par une bride 21 passant sous un relief anatomique connu de la vertèbre. Dans le cas de la figure 5, la bride 21 passe sous la transverse 3a de la vertèbre 3, et s'engage dans la rainure transversale externe 20. La bride 21 est une bande conformée pour être serrée en anneau, de façon connue, et dont la largeur est peu inférieure à la largeur de la rainure transversale externe 20, pour éviter les mouvements relatifs entre la bride 21 et le moyen d'ancrage principal 1 le long de la branche transversale de liaison 6.

On voit que l'orientation oblique de la branche transversale de liaison 6 permet d'engager la tige 5 du clou pédiculaire dans le pédicule 3d de la vertèbre 3, tout en plaquant la branche transversale de liaison 6 sur la transverse 3a de la vertèbre 3, favorisant la stabilité de l'ancrage.

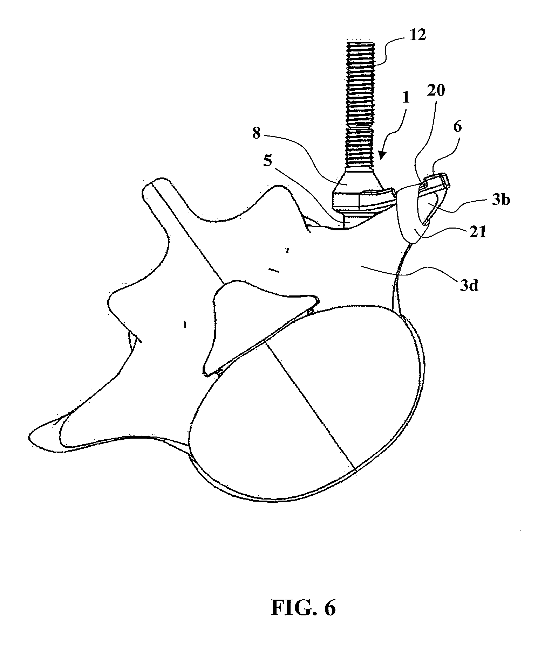

Dans la variante illustrée sur la figure 6, le même moyen d'ancrage principal 1 de la figure 4 est stabilisé par la lame 3b de la vertèbre 3. Dans ce cas, la branche transversale de liaison 6 est orientée pour être plaquée sur la lame 3b, et la bride 21 passe sous le relief anatomique constitué par la lame 3b et s'engage dans la rainure transversale externe 20 de la branche transversale de liaison 6. A nouveau, l'orientation oblique de la branche transversale de liaison 6 est bien adaptée pour que la branche transversale de liaison 6 se plaque sur la lame 3b de la vertèbre 3, tandis que la tige 5 du clou pédiculaire est correctement engagée dans le pédicule 3d de la vertèbre 3.

Lors de la pose, en cours d'opération, le chirurgien peut ainsi choisir le meilleur mode de stabilisation du moyen d'ancrage principal 1, en fonction de l'état de l'os du patient.

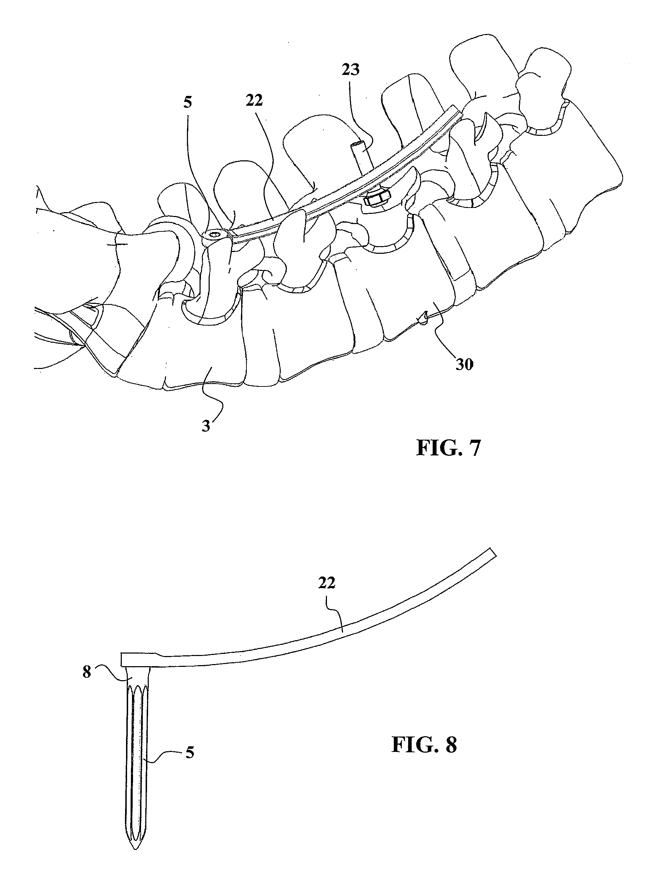

On considère maintenant les figures 7 et 8, qui illustrent un troisième mode de réalisation de stabilisation d'un moyen d'ancrage principal à clou pédiculaire selon la présente invention.

Dans ce cas, la tige 5 du clou pédiculaire, fixée dans une vertèbre 3, est couplée à une tige précintrée 22, soit par un dispositif de connexion de type vis, soit par le fait que la tige précintrée est monobloc avec la tige 5 du clou pédiculaire. Cet ensemble clou pédiculaire 5 - tige précintrée 22 peut être ensuite connecté à une vis pédiculaire 23 insérée dans un pédicule d'une vertèbre adjacente 30, pour assurer une ostéosynthèse intervertébrale courte entre deux vertèbres voisines 3 et 30.

Ce même mode de réalisation peut être utilisé comme moyen d'ancrage sur le sacrum. Dans ce cas, on remplace la tige précintrée 22 par une plaque sacrée qui possède à chacune de ses extrémités un alésage. L'alésage supérieur est oblong et permet le passage d'une vis osseuse comme moyen d'ancrage complémentaire. En outre, l'alésage supérieur a une forme en rampe qui entraîne une traction axiale de la plaque vers le haut lors du serrage de la vis osseuse. L'alésage inférieur est oblique, orienté vers le haut et en dehors, et taraudé. Le moyen d'ancrage principal est un clou, engagé et vissé dans l'alésage inférieur oblique. Le clou est engagé dans l'aileron sacré. La vis est engagée dans le plateau du sacrum. Le serrage de la vis osseuse provoque l'arc-boutement du clou et son plaquage latéral contre l'os dans l'aileron sacré pour s'opposer à son extraction axiale en cas d'efforts d'arrachement.

Les modes de réalisation illustrés sur les figures montrent à chaque fois la stabilisation d'un clou pédiculaire soit par un autre clou pédiculaire orienté obliquement, soit par une bride.

On peut également imaginer, sans sortir du cadre de l'invention, stabiliser un clou pédiculaire à la fois par la connexion à un second clou pédiculaire comme illustré sur la figure 3, et par une bride de stabilisation 21 engagée sur un relief osseux de vertèbre et sur la branche transversale de liaison 6 comme illustré par les figures 5 et 6, et aussi simultanément par une tige précintrée 22 comme illustré sur les figures 7 et 8.

La présente invention n'est pas limitée aux modes de réalisation qui ont été explicitement décrits, mais elle en inclut les diverses variantes et généralisations contenues dans le domaine des revendications ci-après.

## Revendications

1. - Dispositif d'ancrage vertébral, comprenant un moyen d'ancrage principal (1) et un moyen d'ancrage complémentaire (1a, 21) conformés pour s'ancrer chacun par voie postérieure respectivement à une vertèbre (3) et pour être connectés mécaniquement l'un à l'autre, **caractérisé en ce que** :
- le moyen d'ancrage principal est une pièce rigide, comportant un clou pédiculaire à tête (8) proximale et à tige axiale (5) conformée pour être engagée en force dans un canal intérieur d'un pédicule de vertèbre, comportant une branche transversale proximale de liaison (6), et comportant des moyens de connexion (10, 11) pour sa connexion à une tige de réduction (16, 16a) apte à relier plusieurs dispositifs d'ancrage vertébral,
- la branche transversale de liaison (6) du moyen d'ancrage principal (1) est munie de moyens de solidarisation (7, 20) pour sa solidarisation au moyen d'ancrage complémentaire (1a, 21),
- le moyen d'ancrage complémentaire (1a, 21) est conformé pour s'opposer à l'arrachement axial de la tige (5) de clou pédiculaire du moyen d'ancrage principal (1) hors du pédicule.

2. - Dispositif d'ancrage vertébral selon la revendication 1, **caractérisé en ce que** la tige (5) du clou pédiculaire a un corps tronconique dont le diamètre va en se réduisant progressivement à partir de la tête (8) à laquelle se relie la branche transversale de liaison (6).

3. - Dispositif d'ancrage vertébral selon l'une des revendications 1 ou 2, **caractérisé en ce que** la tête (8) comporte un alésage taraudé axial (10), débouchant à l'opposé de la tige (5) du clou pédiculaire, pour l'insertion d'une tige filetée de connexion (12).

4. - Dispositif d'ancrage vertébral selon l'une quelconque des revendications 1 à 3, **caractérisé en ce que** la tête (8) comporte un alésage taraudé latéral (11), débouchant à l'opposé de la branche transversale de liaison (6), avantageusement selon une orientation (A) de 90° ou 120° par rapport à l'axe (I) de la tige (5) du clou pédiculaire, pour l'insertion d'une tige filetée de connexion (12).

5. - Dispositif d'ancrage vertébral selon l'une des revendications 3 ou 4, **caractérisé en ce que** la tige filetée de connexion (12) comporte un premier tronçon fileté apte à se visser dans l'un des alésages taraudés (10, 11) de la tête (8), une portion centrale (13) à portée extérieure conique (14) pour la stabilisation d'une tige de réduction (16, 16a), et un second tronçon fileté (15) de fixation de la tige de réduction (16, 16a).

6. - Dispositif d'ancrage vertébral selon la revendication 5, **caractérisé en ce que** la tête (8) comporte une surface externe (9) sphérique, et la portion centrale (13) de tige filetée de connexion (12) comporte une portée intérieure sphérique s'adaptant sur la surface externe (9) sphérique de la tête (8).

7. - Dispositif d'ancrage vertébral selon l'une des revendications 1 ou 2, **caractérisé en ce que** la tête est solidaire d'une tige filetée de connexion (12) orientée à l'opposé de la tige (5) du clou pédiculaire.

8. - Dispositif d'ancrage vertébral selon l'une quelconque des revendications 1 à 7, **caractérisé en ce que** la branche transversale de liaison (6) est galbée de façon à épouser le profil de l'arc postérieur de la vertèbre (3) sur laquelle elle doit être implantée.

9. - Dispositif d'ancrage vertébral selon la revendication 8, **caractérisé en ce que** les moyens de solidarisation comprennent un trou (7) dont l'axe (11) est généralement parallèle à l'axe (1) de la tige (5) du clou pédiculaire.

10. - Dispositif d'ancrage vertébral selon la revendication 9, **caractérisé en ce que** deux moyens d'ancrage principaux (1, 1 a) sont solidarisés l'un à l'autre de façon rigide par un ensemble vis-écrou engagé dans des trous (7) correspondants des branches transversales de liaison respectives (6, 6a) orientées l'une vers l'autre et venant à recouvrement partiel l'une de l'autre, les tiges (5, 5a) des clous pédiculaires étant orientées selon deux orientations convergentes se rapprochant l'une de l'autre vers leurs extrémités libres.

11. - Dispositif d'ancrage vertébral selon l'une quelconque des revendications 1 à 7, **caractérisé en ce que** les moyens de solidarisation du moyen d'ancrage principal (1) comprennent une rainure transversale externe (20).

12. - Dispositif d'ancrage vertébral selon la revendication 11, **caractérisé en ce que** le moyen d'ancrage complémentaire est une bride (21) apte à entourer à la fois un relief osseux de la vertèbre (3) et la branche transversale de liaison (6) du moyen d'ancrage principal (1) en s'engageant dans la rainure transversale externe (20).

13. - Dispositif d'ancrage vertébral selon l'une quelconque des revendications 1 à 7, **caractérisé en ce que** la branche transversale de liaison (6) du moyen d'ancrage principal (1) est une tige précintrée (22) monobloc ou rapportée, apte à être connectée à une vis pédiculaire (23) pour assurer une ostéosynthèse intervertébrale courte.
